# EUROPEAN PATENT SPECIFICATION

(11) **EP 4 491 207 B1**
(45) Date of publication and mention of the grant of the patent: **04.11.2026**
(21) Application number: 24210169.9
(22) Date of filing: 16.09.2022
(51) Int. Cl.: A61M 5/24, A61M 5/28, A61M 5/31

(54) **SYRINGE SYSTEM**
SPRITZENSYSTEM
SYSTÈME DE SERINGUE

(30) Priority: 16.09.2021 GB 202113208; 30.10.2021 GB 202115625; 18.11.2021 GB 202116639; 01.12.2021 GB 202117335; 14.04.2022 GB 202205526; 15.07.2022 GB 202210475
(43) Date of publication of application: 15.01.2025
(62) Document number(s) of the earlier application(s) in accordance with Art. 76 EPC: 22792785.2 / 4 376 921
(73) Proprietor: Dunne, Stephen Terence, 4150-044 Porto (PT)
(72) Inventor: Dunne, Stephen Terence, 4150-044 Porto (PT)
(74) Representative: Tergau & Walkenhorst Intellectual Property GmbH

(56) References cited:
- WO-A1-2015/055592
- US-A- 2 865 372
- US-A- 4 445 895
- US-A- 4 834 717
- US-A1- 2012 041 384
- US-A1- 2015 073 352

## Description

The invention relates to a syringe system, in particular a manual syringe system.

Glass and plastic pre-filled syringes or ready-to-fill syringes are supplied for use with the drug formulation already in the syringe and ready to use. There are two formats. The first has a Luer connection for attaching a hypodermic needle. The second format, the staked needle syringe, has the needle already attached. Both formats need to be lubricated with silicone oil to facilitate stopper or piston travel.

Both have advantages and disadvantages. The principal advantage of the Glass Luer type is that the silicone lubrication can be baked on giving it superior lubrication and the choice of needle is unlimited. The biggest disadvantage is that the needle needs to be attached by the user which can lead to user errors and even loss of sterility.

Glass staked needle syringes come with the needle attached and held in place which is its biggest advantage. The disadvantages are that the needle, epoxy glue and residue tungsten from its manufacture are in touch with the drug formulation during storage and that the needle tip can be damaged or blunted by the needle guard because the needle is embedded into the rubber to seal it. Additionally, silicone lubrication oil cannot be baked on because of the epoxy glue so staked needle pre-filled syringes must rely on inferior sprayed on silicone where the silicone can migrate during storage into the dug causing drug agglomeration or migrate between areas on the glass surface leaving areas unlubricated.

Plastic pre-filled syringes have sprayed on silicone only as the silicone cannot be baked on due to the required high temperatures.

Pre-filled syringes are generally filled with an air space of gas bubble because they are filled from the open end opposite the needle end and then stoppered. Gas bubbles can cause stopper movement and loss of sterility during transport due to pressure changes. Additionally, the head space allows the drug to move around and strip the silicone oil from the surface. Silicone oil droplets can additionally interact with drug molecules and especially biologics. Pre-filled syringes may be filled bubble free by using vacuum stoppering. However, this is generally limited to low viscous formulations and it greatly slows down the filing line adding cost.

Staked needle pre-filled syringes have limited needle options and are available only in ready to fill or ready to use formats. These are expensive compared to bulk formats.

It is preferably where possible that filled syringes are gas terminally sterilized with the drug in place. Generally staked needle pre-filled syringes whether glass or plastic cannot be terminally sterilized because the needle guard that seals the needle tip is made from permeable rubber compounds in order that the needle can be gas sterilized in production before filling. Terminal sterilization by gas would degrade the drug. Luer lock pre-filled syringes can be terminally sterilized, but the user needs to remove the syringe from the sterile packaging before attaching the needle which can lead to a loss of sterility.

Terminal sterilization of a pre-filled syringe is potentially a big advantage in most settings as it much minimizes the risk of cross contamination and loss of sterility. As an example, Luer lock syringes are used in ophthalmologics where the syringe is preferably terminally sterilized. However, the user still needs to attach the needle after removing the syringe from its sterile protective blister leading to potential user errors and a risk of loss of sterility.

An alternative to pre-filled syringes are cartridges-based syringes or pens. The cartridge comprises of a glass or plastic barrel, a pierceable septum held by a crimped metal ferrule at one end and a stopper/piston at the other. Usually, an injection needle is manually attached by the user before use by screwing a double ended needle on to a cartridge holder or other device such as a pen holding the cartridge. This perforates or pierces the septum making a fluid pathway between the cartridge drug contents and the injection needle. Such arrangements are typically used in pen injectors such as insulin pens and cartridge-based dentistry syringes. Because the user needs to attach the needle there is the danger of user errors and a risk of loss of sterility.

Cartridges are available in both ready to fill and much cheaper bulk formats. They can be filled like syringes by capping, filing, and stoppering or bubble free by stoppering, filling and caping.

**It** is an object of the present invention to provide a syringe system that overcomes the problems of both types of syringes and current cartridge-based syringes.

**In** accordance with the invention, this objective is achieved with a syringe system as defined in claim 1.

Preferred embodiments of the invention are defined in the dependent claims.

**In** particular a syringe system in accordance with the present invention comprises of an:
outer casing or syringe housing holding,
a cartridge having a glass or plastic barrel or generally tubular barrel, a needle pierceable septum or sealing element held by a crimped metal ferrule disposed at the distal end of the barrel and a stopper/piston disposed at the proximal end of the barrel to contain the medicament or other formulation between the stopper/piston member and the septum or sealing element,
a plunger rod in communication with the cartridge stopper/piston.

**In** one embodiment there is a fixed septum needle and two position cartridge,
the septum needle is rigidly held in the syringe housing,
the filled cartridge is assembled and held in the outer casing in a first position and the septum needle is in fluid communication with either an injection needle or a transfer nozzle or a connector such as a tip, catheter tip, coiled tip, eccentric tip, intravascular line connector, Luer slip or lock or a threaded connector including means to connect an injection needle and arranged so that the user can push the cartridge relative and towards to the septum needle into a second position where the septum needle perforates the cartridge septum making the syringe ready for use.

In another embodiment the filled cartridge is assembled and held axially in a fixed position with a needle arrangement or hub comprising a septum needle and an injection needle in fluid communication with one another rigidly held in a hub located at the distal end of the syringe housing and free to move axially within the syringe housing towards the cartridge distal so that when pushed axially towards the cartridge the septum needle perforates the septum establishing fluid communication between the cartridge contents and the injection needle.

In yet another embodiment the filled cartridge is assembled and held axially in a fixed position within the syringe housing has a connector at its distal end capable of holding a needle arrangement comprising of a septum needle and an injection needle where the connector is a threaded connector, a coiled tip, a Luer connector or any other suitable connector where the user attaches the needle arrangement and where the septum needle perforates the septum establishing fluid communication between the cartridge contents and the injection needle.

The cartridge may be inserted before sterilization or by the user.

The syringe system may be sterilized inside a sterile barrier packaging such as a blister with or without a drug filled cartridge.

Alternatively, the drug filled cartridge septum and septum needle and injection needle or nozzle, or connector are isolated from the environment by a seal fixed to the syringe barrel in between the syringe housing and the cartridge barrel and a seal in between a cap and the syringe housing where the seal is either fixed to the cap or to the syringe housing and the syringe is terminally sterilized with the drug filled cartridge in place.

Sterilization may be by gas, vapor or radiation.

Depending on the usage the plunger rod may be firmly attached to the stopper or not.

The plunger rod may have a guide with two or more wings or arms that fits into the cartridge by interference fit so that the plunger rod remains in place and stable during transport or handling where the friction between the plunger rod wings and the internal walls of the cartridge can easily be overcome by the user when pressing the plunger rod during operation.

The plunger rod may be fixed and hinged to the syringe housing to allow axial insertion of the cartridge.

The cartridge may be inserted into the syringe housing axially or radially and held in place by snap fits.

The syringe housing may have in-built snap fits that lock the cartridge in place to prevent it moving backwards relative to the needle or needle attachment in transport or handing or when the plunger rod attached to cartridge stopper is pulled back.

In the embodiment with a fixed septum needle and two position cartridge the cartridge may be held in the first position by a deformable or movable stop on the cartridge shoulder or cap or the cartridge may be further held in the first position by a lock on the cartridge shoulder or cap that is released by activating a switch or button or other means.

The cartridge may be held in the first position by a combination of two or all three of the above methods.

The casing or housing preferably has a finger flange or rings at the proximal end and a plunger rod with a thumb pad or ring in communication with the cartridge stopper or piston arranged so that the user can push the piston or stopper to push the cartridge from the first position to the second position ready for priming and/or injecting and in effect forming a manual syringe system.

In yet another embodiment when the syringe has a full and unused cartridge the distance between the finger pads or rings and the plunger rod finger pads or rings is less than 70mm or less than 65mm or less that 60mm. This is particularly suited to users with small hands. This is achieved by using a cartridge with a larger internal diameter shortening is length for the same volume fill.

The plunger rod may be attached to the stopper if the user needs to pull the stopper back.

In the embodiment with a fixed septum needle and two position cartridge the user simply pushes the plunger rod with sufficient force to move the cartridge from the first position to the second position to overcome the interference fit or friction and or the deformable or movable stop. This moves the cartridge to the second position towards the needle arrangement perforating the septum making the system ready for priming and/or injecting.

In the embodiment with a needle hub the user pushes the hub towards the cartridge septum perforating the septum making the system ready for priming and/or injecting.

If the cartridge is filled bubble free or with only a small bubble the user can simply insert the needle into the injection site and depress or push the plunger rod. This first causes the cartridge to move from a first position at a second position where the septum is pierced and on to the injection itself.

If there is a gas or air bubble and / or the user wants to move the stopper to the correct position for accurate dosing before the injection the user can simply push the plunger rod to move the cartridge from a first position at a second position where the septum is pierced and then prime the device before inserting the needle into the injection site and performing the injection.

In the embodiment with a fixed septum needle and two position cartridge it is preferable that the force to move the cartridge from the first position to the second position towards the needle by pressing the plunger rod is not greatly higher or is similar, or even smaller, than the force required to perform the injection or push the liquid drug formulation out via the needle. In this way there is no sudden decrease in the force required for operation between pushing the cartridge forward from the first position to the second position and priming or injecting. This is particularly so when priming the device including removing any gas or air bubbles if so required. If the force required to move the cartridge forward from the first position to the second position is large compared to the priming force it could potentially lead to a loss of liquid formulation via the injection needle during priming.

In this embodiment the device may have a locking mechanism that holds the cartridge in place before use. If the device has a locking mechanism this needs to be unlocked by a switch or button or other means so that the cartridge can be pushed to the second position by pressing the plunger rod.

In this embodiment the device may include a needle cap to cover the injection needle or connector or nozzle prior to use. The needle cap can be used to lock the cartridge at the first position before use. This locking feature may be part of the cap or the housing in which case the lock is held in the locked position by the cap.

The cartridge may be filled with or without an air or gas bubble. This can be expelled during priming before the injection if required. The cartridge may have markings for accurate dose delivery in which case some of the drug can be expelled before injection.

The whole assembly including the cartridge may be terminally sterilized in a blister or other outer packaging. Depending on the drug type and the packaging and device materials the drug filled system can be sterilized using moist heat (steam), dry heat, gamma radiation or other radiation, ethylene oxide gas, vaporized hydrogen peroxide, chlorine dioxide gas, vaporized peracetic acid and nitrogen dioxide.

If the cartridge is not assembled with the outer casing before sterilization, it can be added later by the user after the user removes the assembled casing and needle arrangement from the sterile packaging or blister. Depending on the packaging and device materials the system can be sterilized using moist heat (steam), dry heat, gamma radiation or other radiation, ethylene oxide gas, vaporized hydrogen peroxide, chlorine dioxide gas, vaporized peracetic acid and nitrogen dioxide.

As mentioned above the whole assembly including the cartridge may be terminally sterilized without a blister by the addition of a sterile needle cap and cartridge seal to keep the needles and cartridge septum sterile. Depending on the drug type and the packaging and device materials the drug filled system can be sterilized using moist heat (steam), dry heat, gamma radiation or other radiation, ethylene oxide gas, vaporized hydrogen peroxide, chlorine dioxide gas, vaporized peracetic acid and nitrogen dioxide. If this is done the outside surfaces will not be sterilized before use.

The cartridge barrel may be made of glass or plastic such as COC or COP and in accordance with ISO 13926-1. The syringe system may be adapted for subcutaneous, intramuscular, intradermal, ophthalmologic, intravascular, intravenous or any other type of injections.

The plunger rod may be attached to the cartridge stopper or not depending on the application. For instance, in eye injections any stopper pull back must be avoided so in this case the plunger rod is not connected to the stopper and can only push the stopper.

In dental injections it is usual to aerate the syringe by pulling the plunger rod. In this case the thumb or finger pad on the plunger rod and the finger flange on the housing may be a finger or thumb rings for easy stopper pull back by the user.

The invention may be used in conjunction with a plunger rod connected to an accurate dosing system or may have an integrated accurate dosing system which is advantageous for very small doses.

The invention may be used in conjunction with a mechanical advantage plunger rod to facilitate the injection of viscous liquids and drugs. The plunger rod may be split by a mechanical advantage mechanism where the plunger rod travels a greater distance than the cartridge stopper when the plunger rod is pushed by the user.

Alternatively, the plunger rod is split by a mechanical advantage mechanism where the plunger rod travels a lesser distance than the cartridge stopper when the plunger rod is pushed by the user. This arrangement is particularly advantageous when delivering large volumes to for instance the blood stream.

The mechanical advantage mechanism may be a cam arrangement.

The double needle arrangement may consist of two separate needles in fluid communication with one another where the septum needle is of larger diameter or smaller gauge than the injection needle to facilitate the injection of viscous drugs.

The double needle arrangement may have two beveled or sharp ends.

The current invention may be used to deliver or inject any liquid into any body including human or animal for any purpose.

Where the injection needle is not supplied attached to the outer casing or syringe housing the syringe housing may have a transfer nozzle or a connector such as a tip, catheter tip, coiled tip, eccentric tip, intravascular line connector, Luer slip or lock or a threaded connector including means to connect an injection needle, or an intravascular infusion tube after removing the device from the sterile packaging. In this arrangement it may be advantageous that the cartridge is held in a first position by a locking mechanism that is unlocked by a switch or button or other means independently from the cap so that the cartridge can be pushed to the second position by pressing the plunger rod only after the user has attached the needle or other connection. A great advantage of this arrangement compared to a Luer lock pre-filed syringe is that when attaching the needle or other connection the fluid in the cartridge is still sealed from the atmosphere as this can be done before moving the cartridge forward to perforate the septum. In this embodiment it is advantageous to keep the cap and lock separate so that the cap can be removed to attach the needle or intravenous or intravascular line before unlocking the cartridge.

The invention may be used in conjunction with a dual chamber cartridge for the reconstitution lyophilized pharmaceuticals or drugs or any other dry powders or the mixing of two pharmaceutical or drug solutions or formulations or any other solutions prior to injection. For mixing the lyophilized drug and the diluent the cartridge is pushed forward to perforate the septum allowing the air in the cartridge to exit through the needle when pushing the plunger rod to mix the drug solution. In this arrangement it may be an advantageous to have a two-position needle cap or a cap and separate switch to release the cartridge. For example, the cap may be rotated to release the cartridge lock and pulled to remove and remain in place protecting the needle until used.

The current invention may be used to deliver or inject the resulting mixed liquid solution from the dual chamber cartridge into any body including human or animal for any purpose. The current invention is suitable for intradermal, subcutaneous, intra-muscular and intravascular injections as any needle type of any gauge and length can be supplied ready to use or attached by the user when the device is supplied with a Luer slip or a Luer lock or a screw type connection. Alternatively, the device may be supplied with a catheter tip or an eccentric tip or any other connector for intravenous or any other intravascular infusion.

In the embodiment with a needle hub the needle cap may be rotatable relative to the syringe body so that it travels axially towards the needle hub pushing the hub towards the cartridge septum and perforating the septum.

In the embodiment with a needle hub the syringe may have a sleeve that can be used to lock the cartridge at the first position before use where the sleeve can be moved by the user axially relative to the outer casing so that it travels axially towards the needle hub pushing the hub towards the cartridge septum and perforating the septum.

The sleeve may have finger flanges that when pulled by the user towards the syringe finger flanges the sleeve travels axially towards the needle hub pushing the hub towards the cartridge septum and perforating the septum.

The syringe sleeve may also be a needle safety guard which is pushed forward by the user towards the distal end of the syringe housing after the injection to cover the injection needle.

The present invention may be used for a single use or single dose where the single dose may be either a single delivery or a number of individual deliveries to generally the same site using the same cartridge and needle. If both the cartridge and injection needle are replaced each time the syringe system may be used for multiple doses and is re-usable.

Environmentally friendly materials may be used to manufacture all or some parts of the syringe system including wood-based materials or bio-degradable plastics.

The current invention is a great improvement over the existing state of the art pre-filled syringes or cartridge injection systems. The drug is stored in a cartridge with the drug only in contact with the barrel material, glass or plastic and an elastomeric material. This is the same as with storage in a vial. Additionally, the cartridge may have the superior baked-on silicone and the whole system with filled cartridge may be terminally sterilized with the injection needle already in place. As far as the user is concerned the current invention is exactly the same as a conventional staked-needle pre-filled syringe with no addition user steps. The invention combines the ease of use of a staked needle pre-filled syringe without its drawbacks. Syringe systems built around cartridges are described in the prior art.

Pertinent prior art is disclosed in US 2012/041384 A1.

US4834717 describes a syringe with a double ended needle fixed to syringe housing holding a cartridge in a first position. The cartridge is held in the first position by a movable stop at the cartridge distal end and by friction between the cartridge and the syringe housing. In the present invention the cartridge can be held in the first position by a lock. In US4834717 there is no tab or lock or stop or shoulder to hold the cartridge at the proximal end. In the present invention there is. With the syringe described in US4834717 there is nothing to stop the user from inadvertently pushing the cartridge forward and perforating the septum before use or pulling the cartridge out of the syringe housing inadvertently. Likewise, sterilization of the double needle and cartridge septum is not considered in US4834717 unlike with the present invention.

US10603439 describes a syringe with a double ended needle fixed to syringe housing holding a cartridge in a first position. The needles and septum are enclosed by seals in a sterile assembly to keep them sterile before use. The cartridge is additionally held axially in a first position only at its distal end by a sterile sealing element. This is extremely complex and extremely difficult to assemble in an aseptic environment and very difficult to prove the parts are sterile. In the present invention a sterile assembly is either not required as the syringe with or without the cartridge is terminally sterilized in a sterile package or the sterile assembly has simple seal arrangement at its needle cap and cartridge barrel and is terminally sterilized and the cartridge is held in an axial position by a simple feature at the proximal end and a simple feature at its distal end.

Additionally, in US10603439 the cartridge is held in a first position using flexible locking tabs at the cartridge distal end that are normally closed and whose resistant needs to be overcome by the user when moving the cartridge from the first position to the second position. In the present invention the tabs if used can be in a normally open position and held by a needle cap or needle sleeve or guard eliminating the need for the user to overcome their resistance. Additionally in the present invention the locking feature may be part of the needle cap and fully removed with cap removal facilitating the injection stroke.

US10561793 describes a syringe with a double ended needle held in a needle hub. To keep the needles sterile in storage the needle hub has an adapter slidingly engaged to the hub that acts as a sterility barrier or seal. Since the needle hub and adaptor need to move axially to perforate the septum the sealing is problematical and extremely complex and extremely difficult to assemble in an aseptic environment and very difficult to prove the parts are sterile. In the present invention the needles are either sterilized in a packaging such as a blister or the sterile seals are fixed and independent from the needle hub so don't move with the needle hub making it far easier to maintain sterility and terminally sterilize. Additionally in the present invention there are sufficient gas pathways to fully terminally gas sterilize the needles and cartridge septum. Where sterile seals are used the gas pathways are covered by the gas permeable needle cap to allow gas in and seal the gas pathways from foreign matter.

US 11065388 describes another cartridge-based syringe system. In this case the distal end of the cartridge holds a complex piston and seal valve system which is not the standard ISO configuration. The present invention uses a well understood needle and septum arrangement.

The prior art above generally describes relatively complex devices that will be difficult to implement, manufacture or assemble. The current invention is a syringe system which is elegant in its simplicity and easy to implement, manufacture or assemble and is an alternative to those currently available but with added advantages.

Various embodiments of the present invention, by way of example, are provided in the figures. Therein,
Figure 1 shows a 'state of the art' standard ISO 13926 Cartridge.
Figures 2 shows the basic invention and prior art.
Figures 3 shows the functioning of the basic invention and prior art with a removable needle cap.
Figures 4 show terminal sterilization options.
Figure 5 shows the prior art with a cartridge holding feature.
Figure 6 shows a further embodiment of the invention with a cartridge holding feature.
Figures 7 show further embodiments of the invention with a cartridge locking feature as part of a needle cap that when removed unlocks the cartridge and releases the cartridge ready for use.
Figure 8 show further embodiments of the invention with a cartridge locking feature as part of the syringe housing with a needle cap that when removed unlocks the cartridge and releases the cartridge ready for use.

In Figures 9 a needle stick injury prevention sleave is shown.

In Figures 10 an integrated precise dosing mechanism is shown.

In Figures 11 various cartridge lock embodiments are shown.

In Figures 12 a dual chamber cartridge option is shown.

Figures 13 show further embodiments of the invention with a cartridge locking sleave with finger pads.

Figures 14 show further embodiments of the invention with a cartridge locking sleave combined with a needle shield or guard with finger pads.

Figures 15 show a further embodiment of the invention with needle hub.

Figures 16 show a further embodiment of the invention with needle hub with a sterile assembly.

Figures 17 show a further embodiment of the invention with needle hub and a needle cap and actuator.

Figures 18 show a further embodiment of the invention with needle hub and a screw off cap and actuator.

Figures 19 show a further embodiment of the invention with a plunger rod incorporating a cartridge mating feature.

Figures 20 shown further embodiments of the invention with cartridge inserted into the syringe housing.

Generally, identical parts are provided with the same reference numbers in all figures. The invention will be explained below primarily with reference to the embodiments shown. However, other embodiments are of course also conceivable and covered by the present invention.

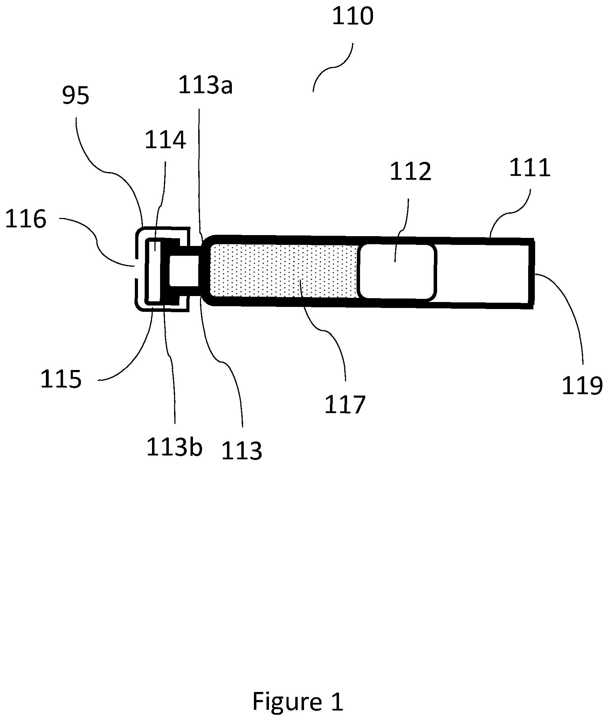

In Figure 1 a cartridge 110 has a plastic or glass barrel 111, an open end 119 and a necked 113 and flanged end 113b at a distal end forming a shoulder 113a with the barrel. A movable stopper plunger 112 at open end 119 and a rubber cap assembly 95 at the necked end or distal end 113 seal the drug formulation solution 117 within the barrel 111. Cap assembly 95 has a septum 114 which is held in place by a metal crimp or ferrule 115 which is in turn crimped to the cartridge neck 113. Ferrule 115 has a hole 116 to allow needle insertion through the septum 114.

Such cartridges are fully described in ISO 13926 and can have glass or plastic barrels.

In the following Figures the cap 95 refers to the combination of a ferrule and a septum assembly as described in Figure 1.

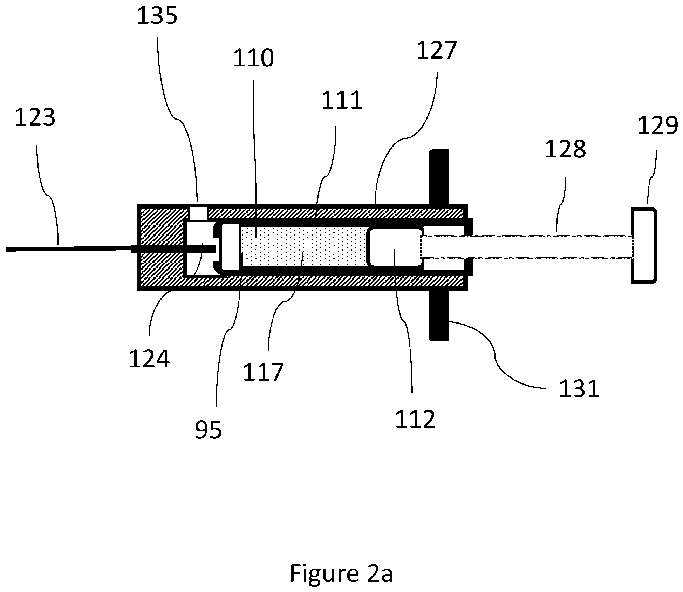

Figure 2a shows the syringe system of the prior art. A cartridge 110 is mounted within a casing or housing 127 at a first position. A double needle comprising an injection needle 123 and a septum needle 124, the two needles in fluid communication, is firmly held at the front end of housing 127. Cartridge 110 is axially free to move under user force in the direction of the septum needle 124 to a second position and when axially pushed it forces septum needle 124 to pierce the cartridge cap 95 enabling the syringe system ready for injecting.

The user can push the plunger rod 128 using the thumb or finger pad 129 and finger flanges 131 in the usual way to axially push the cartridge 110 to a second position to enable the syringe system and prime the system if required and perform the injection of contents 117 via injection needle 123.

Needles 123 and 124 may be separate needles of different gauges or formed from a single needle with two sharp beveled ends. The injection needle may have a blunt end. It may have one or more bends. Needles 123 and 124 may be separate needles and the septum needle 124 may be of a larger diameter or smaller gauge compared to the injection needle 123 to minimize the force required to inject viscous formulations. The two needle ends may have different bevel arrangements. Injection needle 123 may be a nozzle for connection to another device or system or a connector such as a Luer connector for connecting an injection needle. In the prior art plunger rod 128 is firmly attached to stopper 112.

In the current invention gas-access pathways 135 are placed in casing 127 to facilitate gas sterilization of the needle assembly.

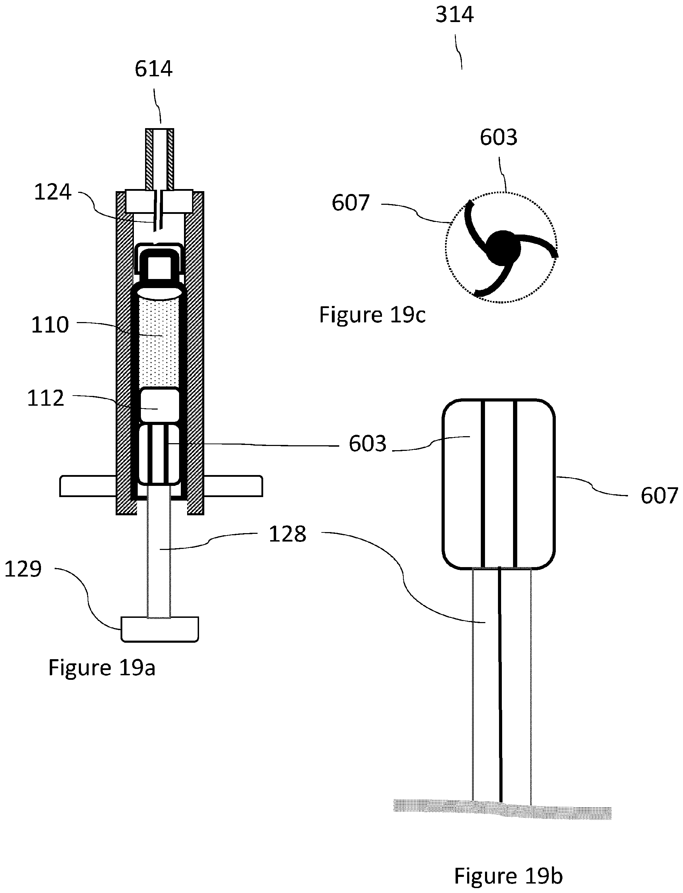

In the present invention the plunger rod 128 may or may not be attached to the stopper 112. Where no plunger pull back is desirable or must be avoided the plunger rod 128 is not attached to stopper 112 and accordingly in the present invention plunger rod 128 may be firmly but slidably held within the cartridge barrel 111 but not attached to the stopper 112. This is shown in Figures 19.

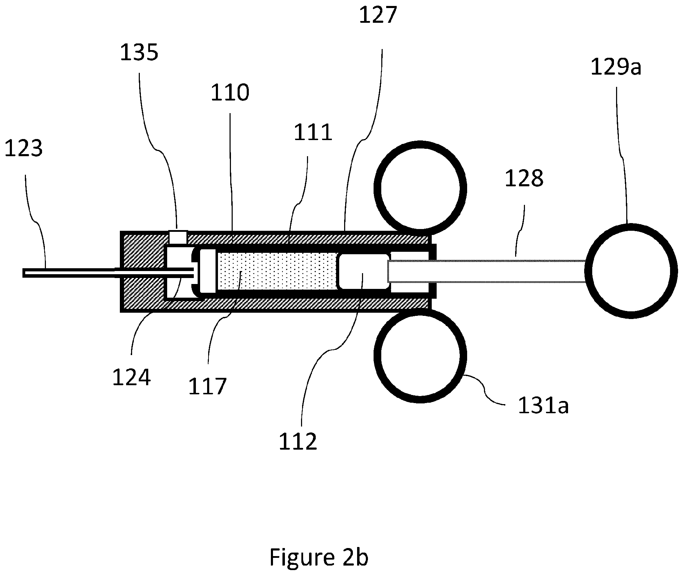

Figure 2b shows the prior art and the present invention with finger pads 129 and 131 of Figures 2a replaced with rings 129a and 131a for easier stopper 112 pull back when required.

Note that in Figures 2 injection needle 123 maybe sized for any application including for subcutaneous, intramuscular or intradermal injections. Additionally, instead of needle 123 a connector or nozzle or other fitting type may be used depending on the requirement including Luer slips or locks for hypodermic needles and catheter tips or eccentric tips for infusion lines or other applications. Unlike in pre-filled syringes in the present invention the connectors or needles are part of the syringe housing not of the primary pack making it easier to supply the syringe with any connector or needle type. Changing primary packs such as syringes or cartridges for different connectors or needles is very time consuming, costly and risky. Not having to do so in the present invention is a great innovation.

Note that casing 127 may be made of a transparent material or have viewing windows or holes in order that the user may inspect the cartridge drug contents before and even during use.

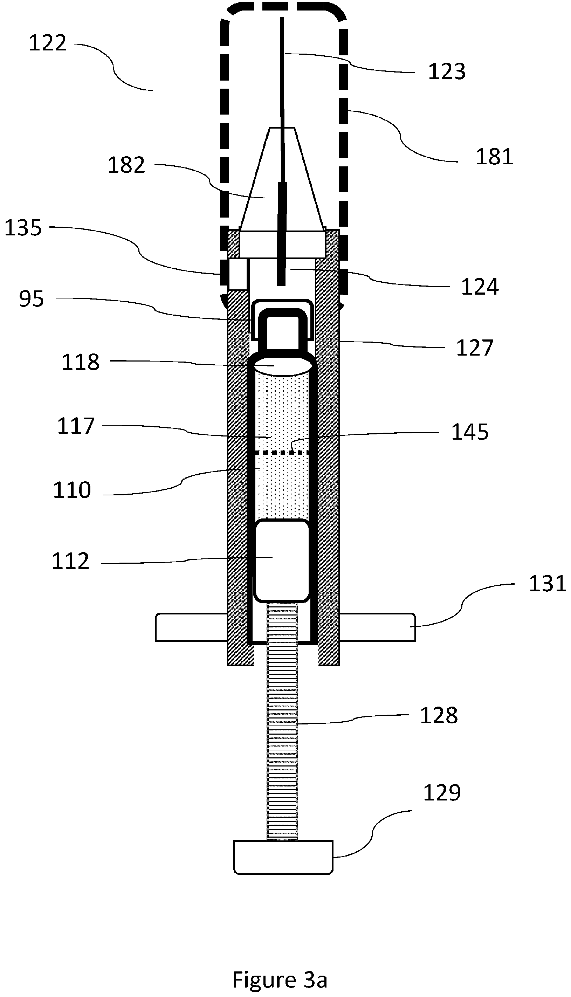

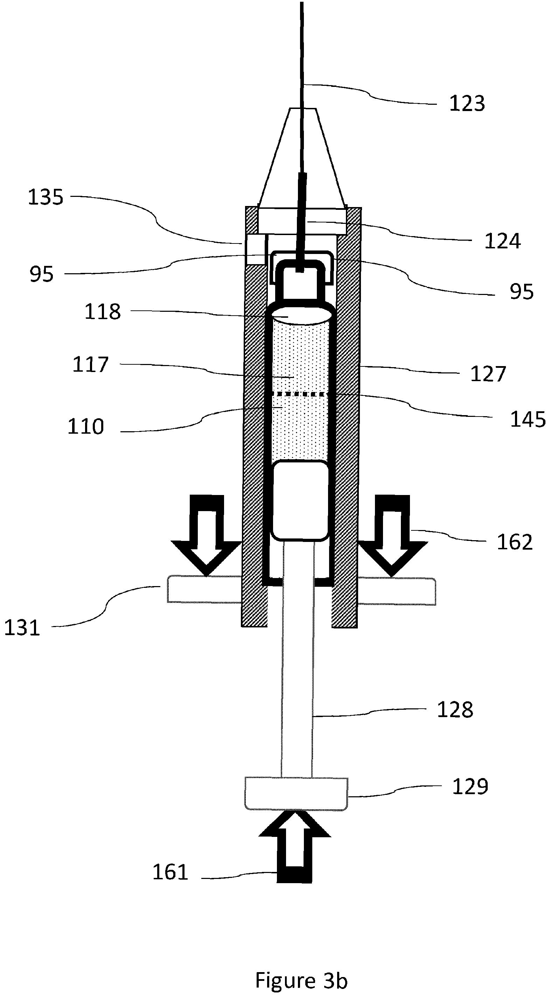

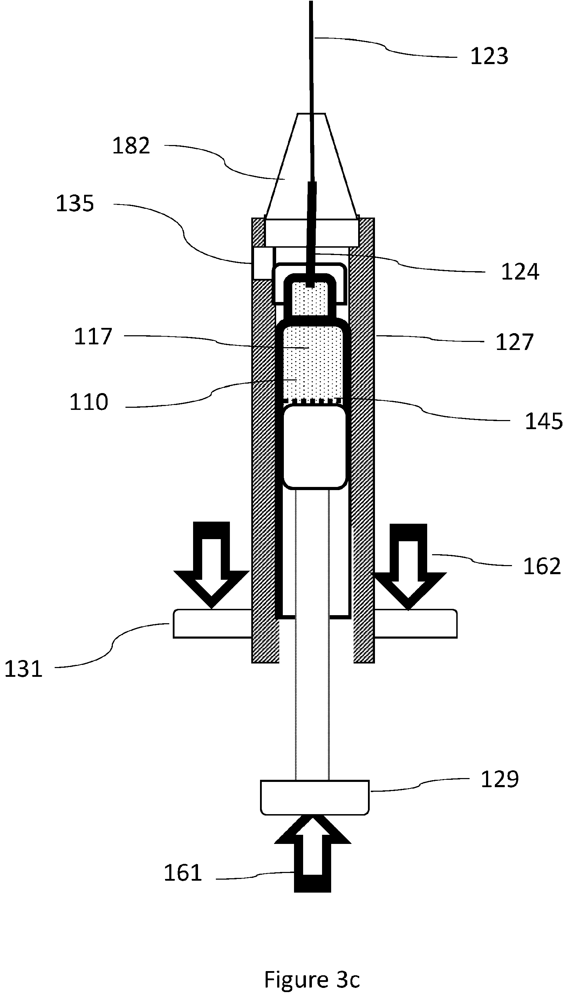

Figures 3 further describe the basic mechanics of the invention and some prior art. In Figure 3a the syringe system 122 has a cartridge 110 free to move axially within casing 127 under user force and is shown in Figure 3a at a first position. In Figure 3b the cartridge is shown in a second position and septum needle 124 has pierced cap 95. To go from a first position shown in Figure 3a to the second position shown in Figure 3b the user uses finger flange 131 and thumb pad 129 in the usual way and pushes/pulls in the direction as shown by arrows 161 and 162 in Figure 3b. In Figure 3c the syringe system has been primed and the stopper/ piston has been moved or primed to the optional dose indication mark 145 before the injection for an accurate volume injection.

Note that the injection can take place all in one stroke with the needle being firstly inserted into the injection site and then the plunger rod pushed. This first pushes the cartridge forwards to perforate septum and is followed by the injection itself. All in one user stroke.

Figures 3a and 3b show an optional gas or air bubble 118 which has been removed after priming in Figure 3c. In some applications an air bubble is advantageous for instance for suspensions. In some other applications it is not desirable.

In Figure 3a an optional cap 181 may be made from gas permeable material or have gas pathways for gas sterilization in a blister and is removed by the user before the injection.

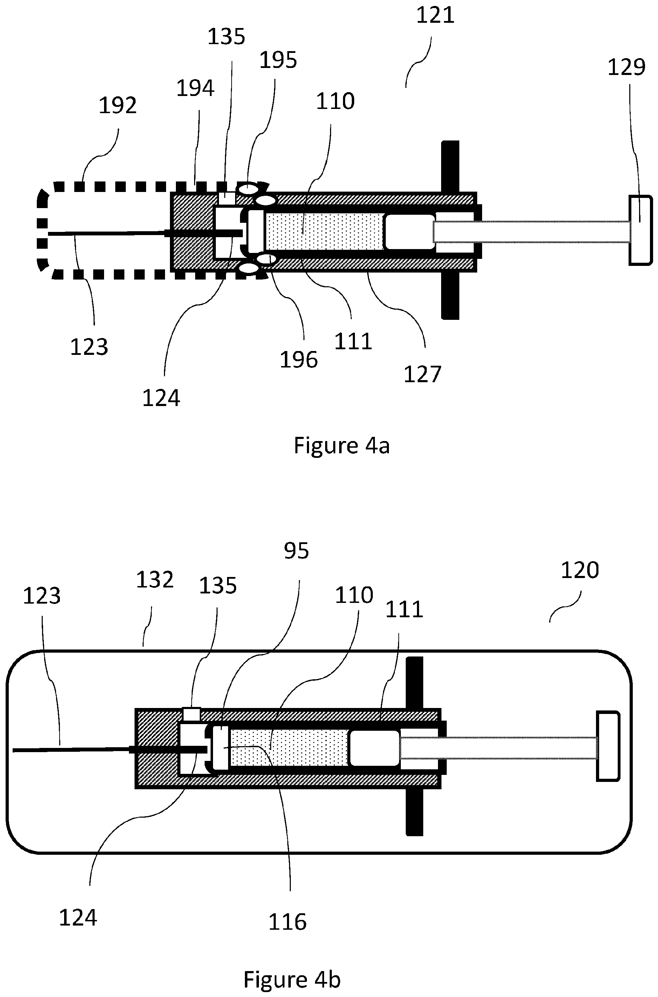

Figures 4 show two options for terminally sterilizing the syringe system of the present invention. The cartridge 110 is shown in a first position.

In Figure 4a the syringe system 121 has a cap 192 or a gas permeable cap 192 for gas sterilization. Cap 192 is sealed against the casing 127 by one or more seals 195 which can be part of cap 192 or part of syringe housing 127. To ensure the needles 123 and 124 and the septum 116 remain sterile after sterilization the barrel 111 of cartridge 110 is additionally sealed against casing 127 by one or more seals 196 which are fixed and securely held in syringe housing 127. Note that gas-access pathway(s) 135 are also coved by the gas permeable cap 192 and that there are sufficient gas pathways to fully terminally gas sterilize the needles and cartridge septum. The gas pathways are covered by the gas permeable needle cap to allow gas in and seal the gas pathways from foreign matter.

Depending on the drug formulation type the system may be sterilized using radiation such as E beam or Gama rays. In this case gas pathways 135 are not required and the cap 192 need not be gas permeable. The great advantage of the present invention when compared to US10561793 are that the seal(s) 196 are fixed to syringe housing 127 and stationary. Additionally, unlike US10561793 no aseptic assembly is required to keep the septum and needles sterile as the whole syringe system in terminally sterilized.

In Figure 4b the syringe system 120 with filled cartridge 110 is placed blister 132 for sterilization and ensure the needles 123 and 124 and the septum 116 remain sterile. If gas sterilization is used a gas permeable blister is used. An optional needle cap may be added but is not shown which if gas sterilization is used must be gas permeable.

Depending on the drug type and the packaging and device materials the drug filled system shown in Figures 4a and 4b may be sterilized using moist heat (steam), dry heat, gamma radiation or other radiation, ethylene oxide gas, vaporized hydrogen peroxide, chlorine dioxide gas, vaporized peracetic acid, nitrogen dioxide or by any other method.

Where gas or vapor sterilization is used there must be sufficient gas pathways to fully gas sterile the needles and cartridge septum. The gas pathways are covered by the optional gas permeable needle cap when used to allow gas in during sterilization.

The syringe system shown in Figures 4b may also be sterilized without the cartridge 110 inserted or in place in which case the cartridge is inserted by the user. It is advisable that the user wipes the cartridge septum with alcohol before assembly.

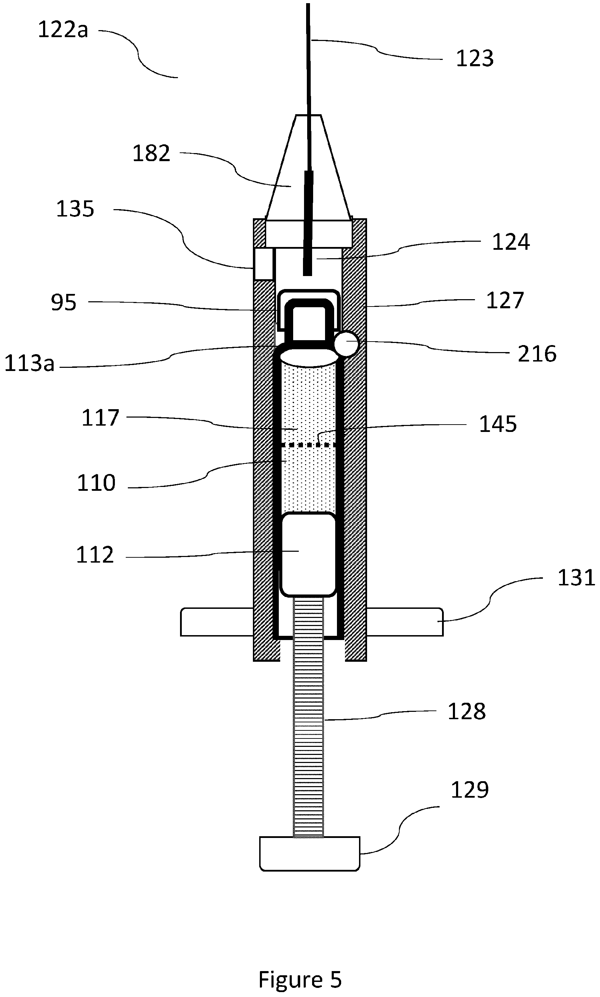

In Figure 5 a means of holding the cartridge in a first position is shown as generally described in the prior art such as US4834717. A syringe 122a has a cartridge 110 with drug solution 117. Cartridge 110 is held in place at a first position by a deformable stop 216. Stops 216 prevents cartridge 110 from moving towards septum needle 124 before the user uses the device by pressing on the plunger pad 129. The deformable stop 216 may be located against the cartridge shoulder as shown in Figure 5 or alternatively against the cartridge cap 95. In US4834717 the stops 216 are hard molded plastic ribs. In the present invention the deformable stops 216 may be an elastomeric 'O' ring or other deformable feature.

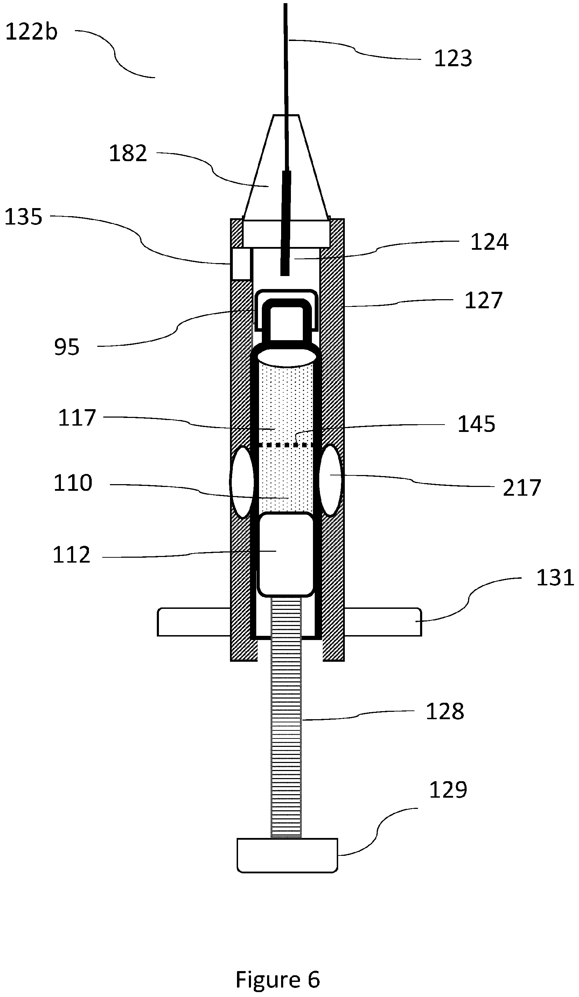

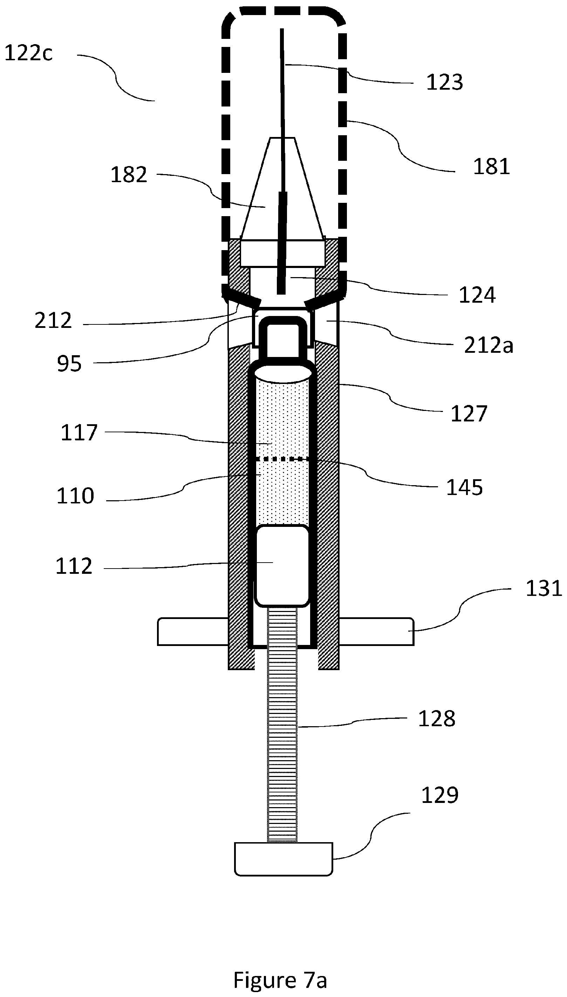

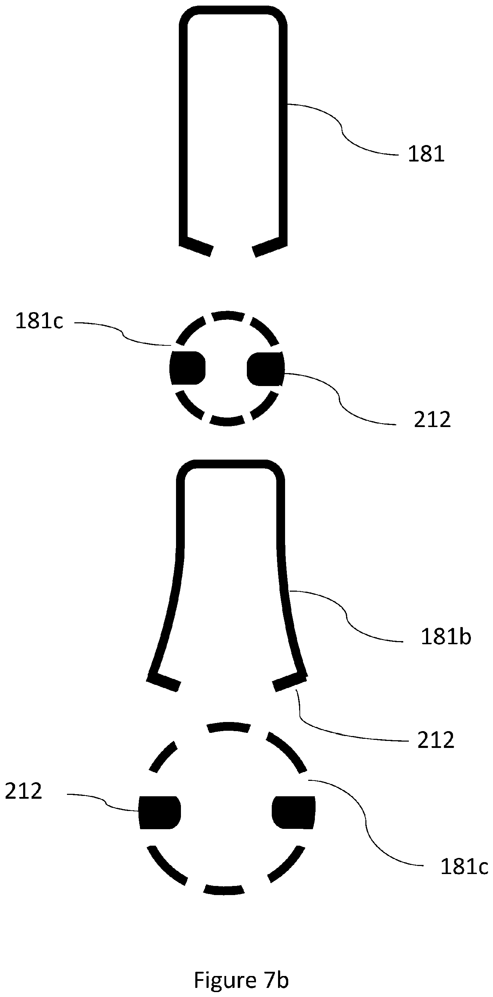

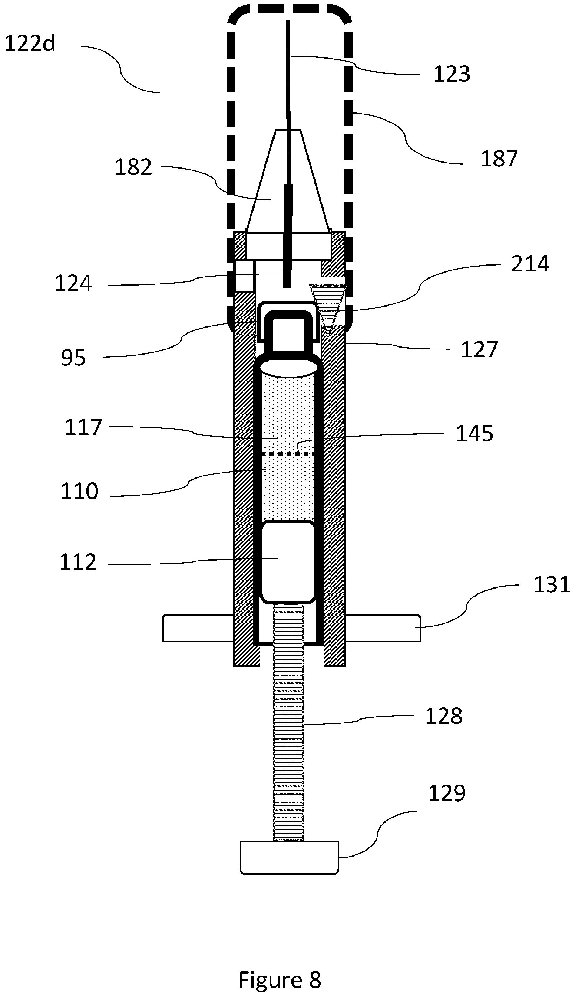

In Figure 6 another means of holding the cartridge in a first position is shown. A syringe 122b has a cartridge 110 with drug solution 117. Cartridge 110 is held in place with one or more friction pads 217. The cartridge is frictionally held in the syringe housing by pads 217 pressing against the cartridge barrel so that when the user presses the plunger rod the force required to move the cartridge towards the needle is similar to the force required to perform the injection or push the liquid drug formulation out via the needle. In this arrangement unlike in arrangement shown in Figure 5 there is no sudden change in the forces required for operation between pushing the cartridge forward and injecting the drug. The friction pads 217 maybe for instance be formed by one or more elastomeric 'O' rings or be molded as part as the syringe housing 127. Such an arrangement can be used in addition to those shown in Figures 7 and 8.

In Figure 7a another embodiment of the present invention is shown. Means of locking the cartridge in a first position is shown. A syringe 122c has a cartridge 110 held in a first position before use by locking tabs 212 formed as part of a needle cap 181 that protrude though through openings 212a in syringe housing 127. Cap 181 may be made of a soft elastomeric material in which case tabs 212 can be formed as part of the cap. Alternatively cap 181 maybe made of a hard plastic with either a soft elastomeric tab 212 attached or tabs 212 may be formed in a hard plastic and hinged and capable of lifting out of the way when cap 181 is pulled off by the user releasing cartridge 110 as additionally shown in Figure 7b.

In Figure 7b a cap 181 has locks tabs 181a. This cap is for use the embodiment shown in Figure 7a. Cap 181 is split into two or more parts by slits 181c. When cap 181 is pulled off by the user the lock tabs 181a bend outwards allowing cap removal. Lock tabs 181a are angled to facilitate removal from the syringe housing.

In Figure 8 a cartridge locking mechanism is shown as generally described in US10603439. A syringe 122d has a hinged lock or flexible locking tabs 214 formed as part of or attached to syringe casing 127 locking the cartridge 110 in a first position before use. Lock 214 is prevented from moving by needle cap 187 before use. Once cap 187 is removed by the locks or tabs 214 can move radially away from the cartridge 110 when the user presses plunger pad 129 allowing the cartridge 110 to move forward to a second position where the needle 124 perforates the cartridge septum ready for injecting.

It must be noted that in the present invention the lock 214 may be alternatively held by a button or a switch or a ring and independently from the cap 187. Additionally, instead of cap 187 removal it may be release by cap 187 rotation or by a ring rotation independently from the cap 187.

In Figure 8 hinged lock 214 maybe have a normally in position or spring loaded and biased towards the locked position as described in US10603439. In the present invention the hinged lock may be of a normally out position or spring loaded and biased towards the unlocked position. Being in the normally out position has the great advantage that the user does not need to overcome the lock with plunger force leading to a smoother injection stroke.

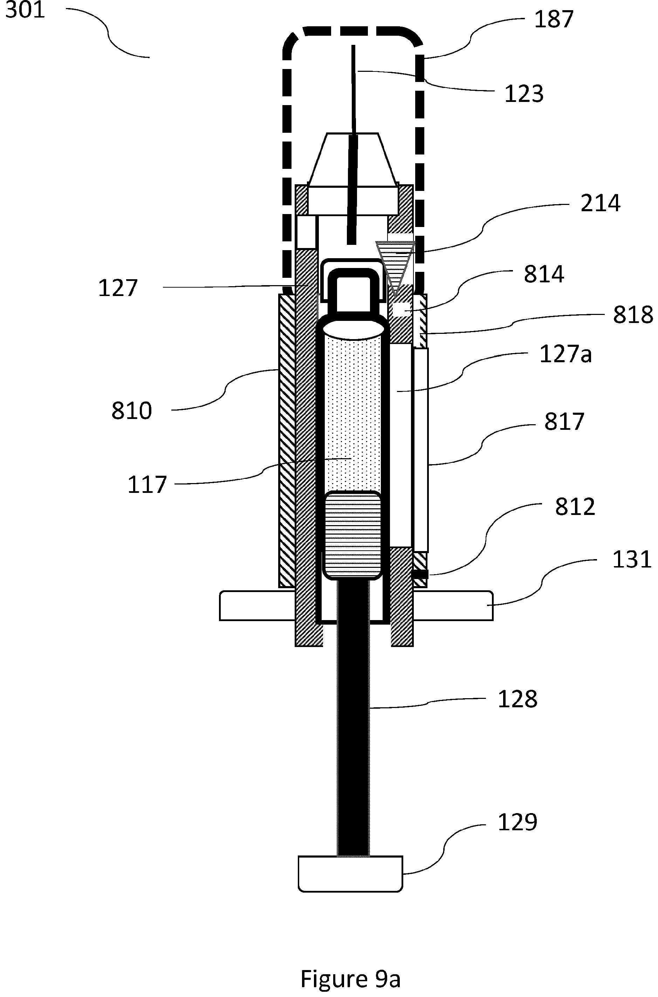

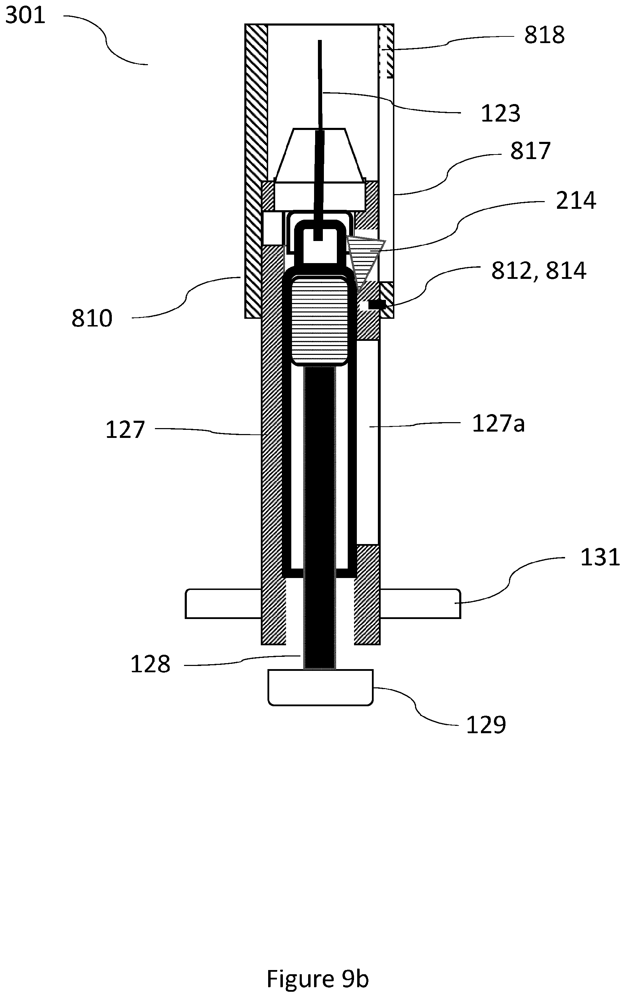

In Figure 9a and 9b another feature of the present invention is shown. Device 301 has a safety sleeve 810 held over a syringe body 127 that is frictionally held in place as shown in Figure 9a before the device is used. A spring-loaded latch 812 is located in the sleeve 810 and this may be used to frictionally hold the sleeve 810 in place before use. Note that the spring-loaded latch 812 may be a simple plastic feature molded as part of the sleeve 810 or a made of separate parts and more than one latch 812 may be used. Sleeve 810 also has at least one viewing window 817 or be transparent allowing the user to inspect the drug formulation before use through viewing windows 127a of syringe housing 127. A groove 818 facilitates the safety sleeve to pass over the lock 214 when pushed by the user to the locked position as shown in Figure 9b

In Figure 9b device 301 is shown after use. Cartridge contents 117 have been expelled via needle 123 by pressing plunger pad 129 and the safety sleeve 810 has been pushed forward by the user covering needle 127 to prevent needle stick injuries after use. Spring loaded latch 812 has snapped into recess 814 in syringe housing 127 locking safety sleeve 810 into place.

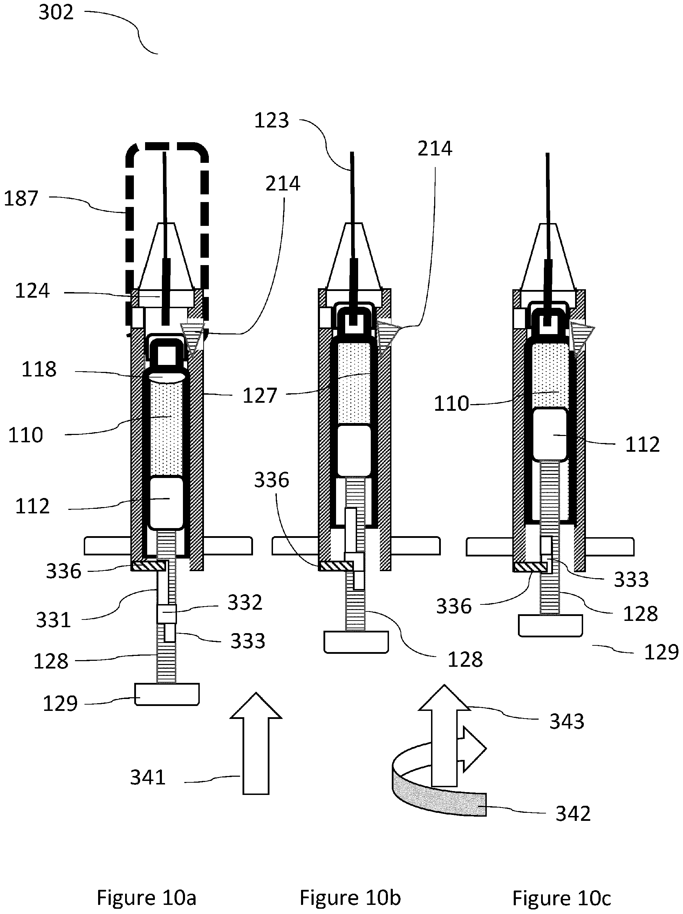

Figures 10 show yet another embodiment of the invention with an accurate dosing system. Device 302 is shown before use in a first position in Figure 10a. It is shown during use with the cartridge in a second position in Figures 10b and 10c. The device of Figures 10 has an accurate dosing mechanism 331, 332, 333, 336 ideally suited for very small doses.

In Figures 10 pin 336 which is attached to body 127 is firstly engaged in slot or groove 331 which is formed in plunger rod 128 as shown in Figure 10a. As plunger rod 128 is pushed towards the cartridge 110 as shown by arrow 341 it moves until pin 336 reaches slot 332 and cannot move further. This second position is shown in Figure 10b. During this movement of the plunger rod 128 the cartridge 110 moves forward from first position to second position and the needle 124 pierces the cartridge septum and the air bubble 118 (if present) and some liquid contents are expelled from device via injection needle 123 priming the device.

In Figure 10c the injection has taken place. To get from Figure 10b to Figure 10c first the plunger rod 128 is rotated as shown by arrow 342 so that pin 336 engages with slot 333. The plunger rod 128 is then pushed as shown in arrow 343. As plunger rod 128 is pushed towards the cartridge 110 as shown by arrow 343 it moves until pin 336 reaches the end of slot 333 and cannot move further administering the exact dose of drug formulation determined by the length of slot 333.

Note that the position of the slots and pin may be reversed with slots 331, 332 and 333 formed in casing 127 with the pin 336 forming part or attached to plunger rod 128. More than one injection slot may be used with a rotation between them so that more than one exact dose of drug can be administered making a multi-dose syringe.

If the cartridge is filled without an air or gas bubble and priming is not required, the first slot 331 can be used only for moving the cartridge forward to the second position ready for injection.

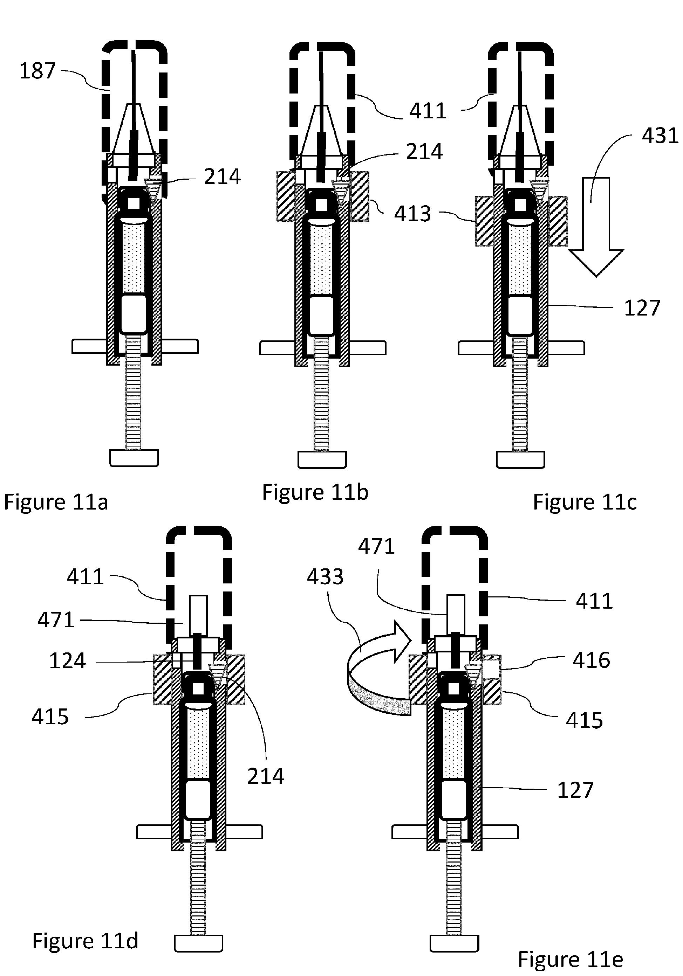

Figure 11 shows various alternative embodiments where the cartridge lock 214 is held in the locked position by a ring 413, 415 instead of the cap 187. Figure 11a shows the same embodiment as Figure 8 where the cap 187 prevents the lock 214 opening. Figures 11b to 11e show alternatives. In Figure 11b the cartridge lock 214 is prevented from opening by ring 413 and not by cap 411. In Figure 11c the ring 413 has been moved down in the direction of arrow 431 freeing the cartridge lock 214.

In Figure 11d again the cartridge lock 214 is prevented from opening by ring 415 and not by cap 411. In Figure 11e ring 416 has been rotated as shown by arrow 433 aligning hole 416 in ring 415 freeing lock 214. In Figures 11d and 11e the injection needle has been replaced by a Luer or other connector 471 in fluid communication with septum needle 124. This can be a Luer slip or a Luer lock connection for a hypodermic needle or a catheter tip or an eccentric tip or any other connector for intravenous infusions or other uses.

The ring 415 of Figure 11d and 11e may be part of cap 411 in which case the cap 411 is rotated to release the cartridge and pulled to expose the injection needle, Luer or other connector.

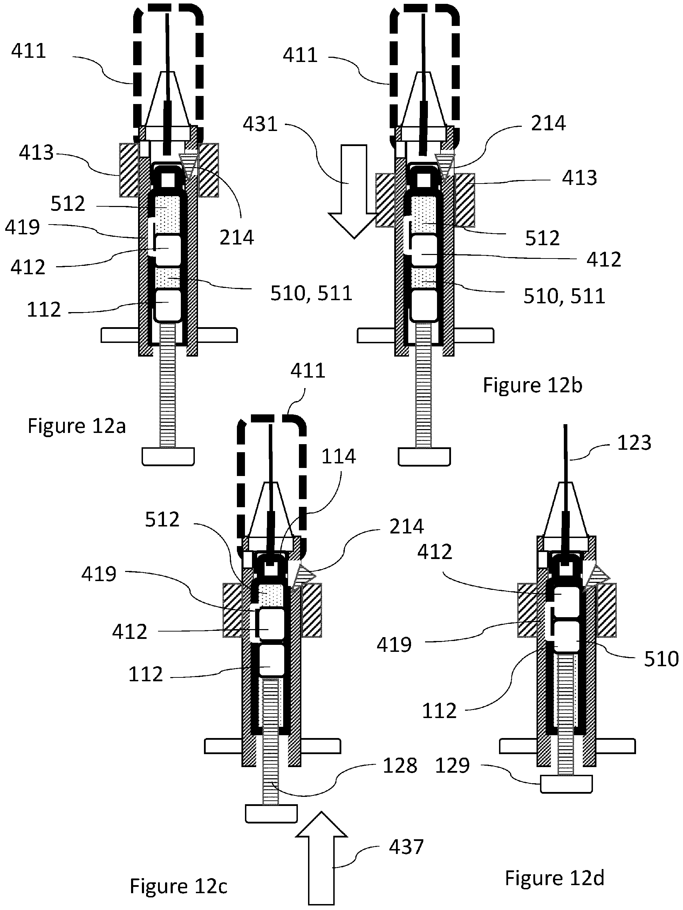

The arrangement shown in Figures 11 is particularly suited for use with a double chamber cartridge. This is shown in Figures 12.

In Figure 12a the syringe has a dual chamber cartridge 510 which has two chambers divided by stopper 412. Chamber 511 contains a dilutant or a liquid drug formulation while chamber 512 contains a dry powder drug or lyophilized drug or a liquid drug formulation. Cartridge lock 214 is prevented from opening by ring 413. A bypass 419 connects the two chambers and is blocked or closed by stopper 412.

In Figure 12b the ring 413 has been moved down in the direction of arrow 431 freeing the cartridge lock 214.

In Figure 12c the plunger rod 129 has been pressed as shown in arrow 437. Cartridge 510 has moved forward and the cartridge septum has been perforated. Further pushing plunger rod 129 has moved stopper 112 forward. Because of the liquid stored in chamber 511 stoper 412 has also moves forward opening bypass 419 forcing the liquid in chamber 511 to enter chamber 512 mixing with the contents of chamber 512. Any air trapped in chamber 512 has been forced out via the needles.

In Figure 12d the cap 411 has been removed and the mixed contents in chamber 512 have been injected via injection needle 123.

The cartridge locking may be done also by a cap that is first rotated to unlock cartridge before removing it for the injection. Ring 413 can also be rotated instead of pulled down to release the lock.

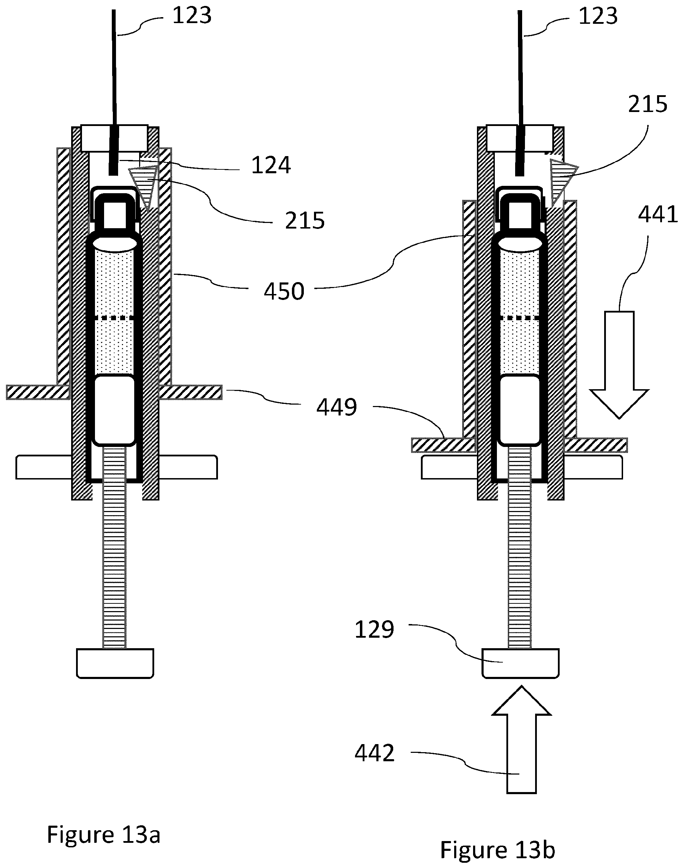

In Figures 13 another embodiment of the present invention is shown where a lock 215 is held in place by a sleave 450 that has finger pads or flange 449. To unlock the cartridge and proceed with the injection finger pads 449 and 129 are respectably pulled and pushed as shown by arrows 441 and 442.

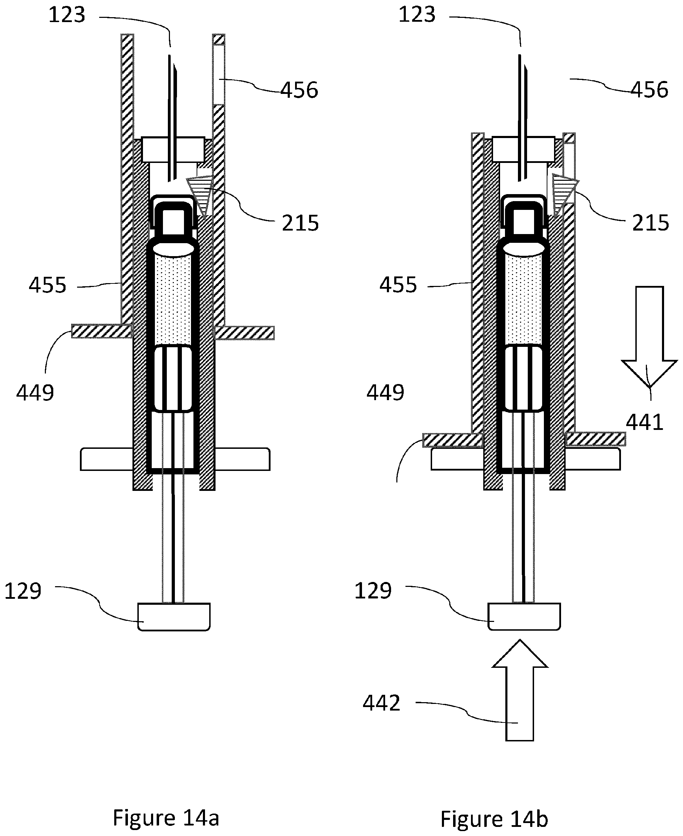

In Figures 14 another embodiment of the present invention is shown where a lock 215 is held in place by a sleave and needle shield or cap 455 that has finger pads or flange 449. To unlock the cartridge and proceed with the injection finger pads 449 and 129 are respectably pulled and pushed as shown by arrows 441 and 442 as shown in Figure 14b. Shield 455 may also be a needle safety guard and can be moved back and locked into place to cover the needle after the injection.

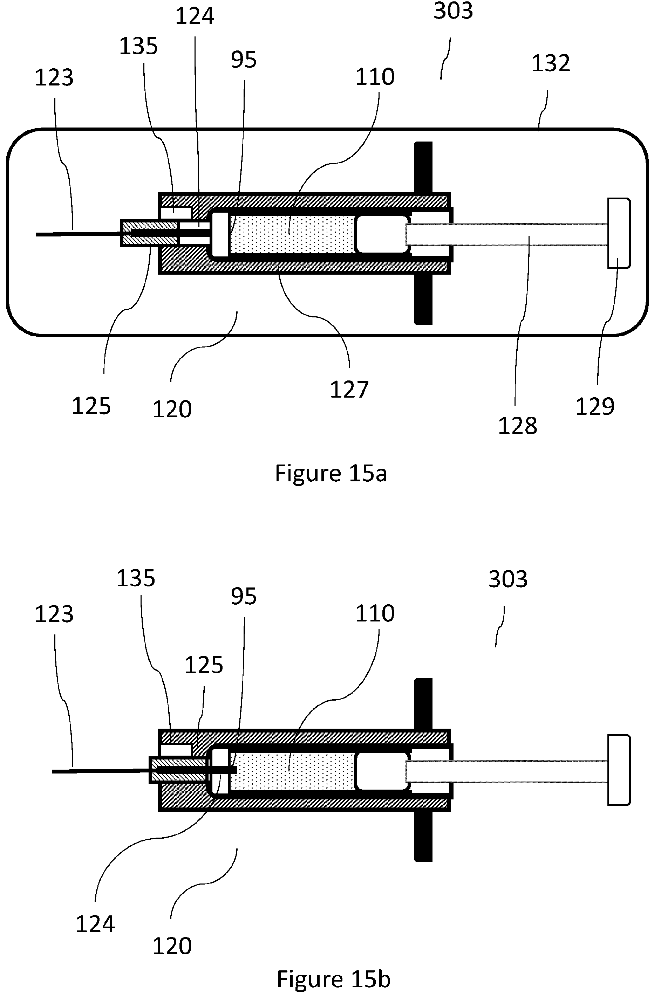

Figures 15 show the prior art as generally described in US10561793 but without the complex sterile assembly shown in US10561793.

In Figure 15a syringe system 303 has a syringe housing 127 rigidly holding cartridge 110 within. Cartridge 110 has a cap assembly 95 at the distal end and a plunger rod 128 with thumb pad 129 at the proximal end. Attached to the syringe housing and opposite the cartridge cap 95 and free to move axially within is a needle hub 125 holding a septum needle 124 and an injection needle 123 in fluid communication with one another.

According to the present invention the syringe system shown in Figure 15a is sterilized in a sealed blister 132 with or without the filled cartridge in place. For gas sterilization gas pathways 135 facilitate the circulation of gas during sterilization.

Figure 15b the syringe has been removed from the blister 132 and the injection has taken place. The hub 125 has been moved towards the cartridge, the cartridge septum has been pierced by the septum needle 124 and the cartridge drug contents have been delivered through the injection needle 123.

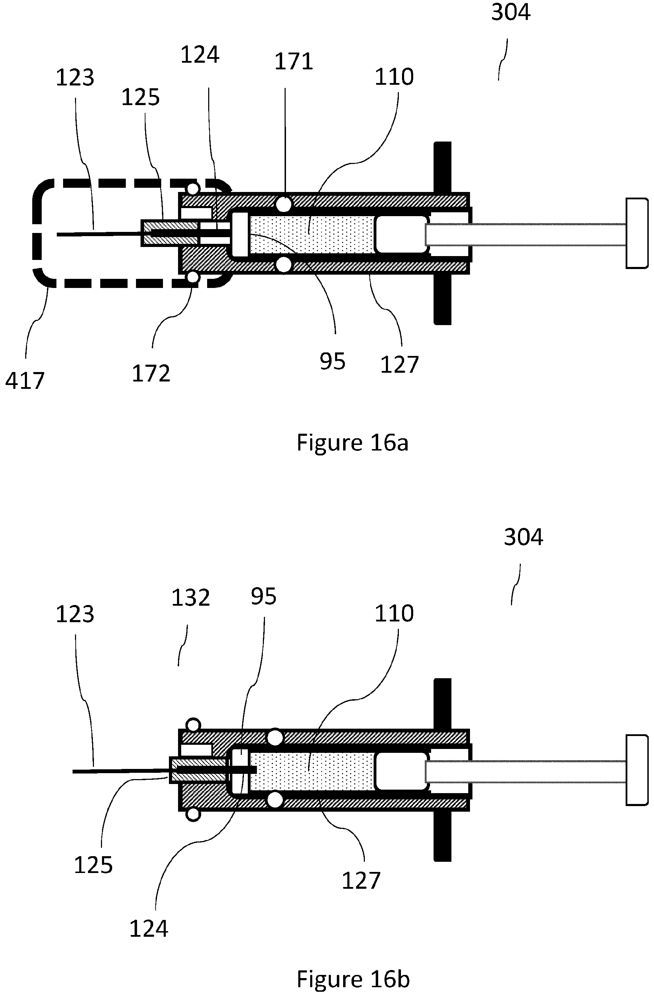

Figures 16 also show the prior art as generally described in US10561793 but with a vital usable improvement with a simple design and where the syringe system is designed to be terminally sterilized.

In Figure 16a syringe system 304 has a syringe housing 127 rigidly holding cartridge 110 within. Cartridge 110 has a cap assembly 95 at the distal end and a plunger rod 128 with thumb pad 129 at the proximal end. Attached to the syringe housing and opposite the cartridge cap 95 and free to move axially within is a needle hub 125 holding a septum needle 124 and an injection needle 123 in fluid communication with one another. Seals 171 between cartridge 110 and syringe housing and seals 172 in between the needle cap 417 and the syringe housing 127 seal the needles and the cartridge septum and keeps them sterile. The syringe system is terminally sterilized. If gas sterilization is used cap 417 is gas permeable.

Figure 16b the needle cap 417 has been removed and the syringe has been used. The hub 125 has been moved towards the cartridge, the cartridge septum has been pierced by the septum needle 124 and the cartridge drug contents have been delivered through the injection needle 123.

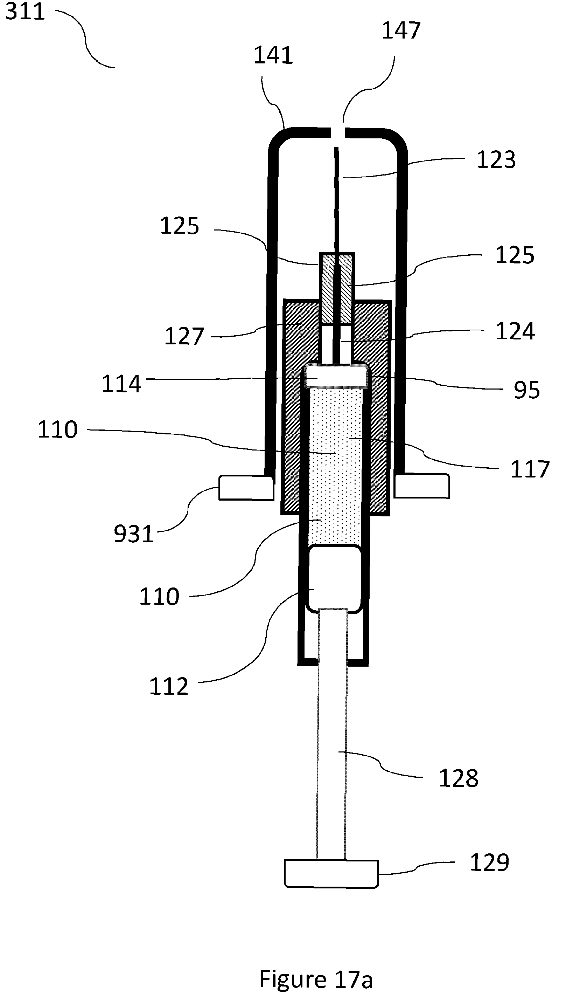

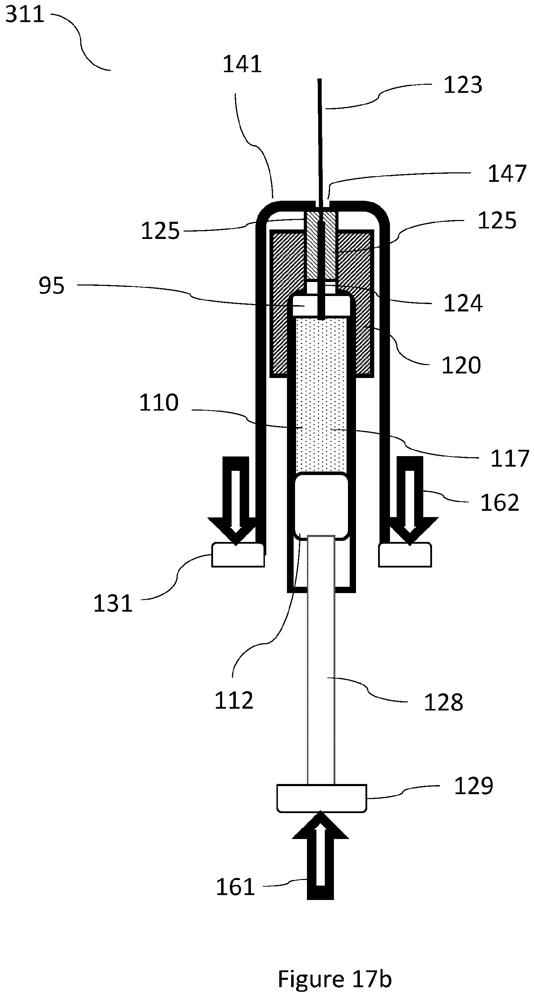

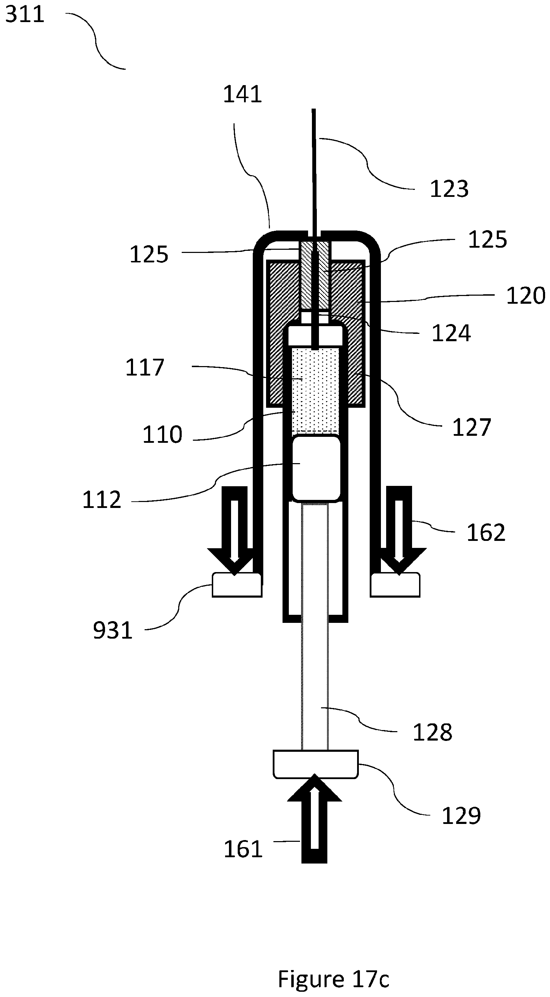

Figures 17 show an embodiment of the invention that facilitates the usage of the syringe systems shown in Figures 15 and 16. In Figure 17a a syringe system 311 has a needle cap and actuator 141 is sleaved over syringe housing 127 covering injection needle 123. The finger pads 931 are attached to actuator 141. Operation is shown in Figure 17b where the user has pressed plunger pad 161 and puled finger pads 931 as indicated by arrows 161 and 162. The needle hub has engaged with actuator 141 and been pushed towards the cartridge and septum needle 124 has perforated cartridge septum ready for injection. Figure 17c shows the syringe system halfway through delivering the cartridge formulation 117.

The needle cap and actuator 141 in Figures 17 may also be used as a safety needle guard and pushed back over the needle and locked into place by the user after the injection.

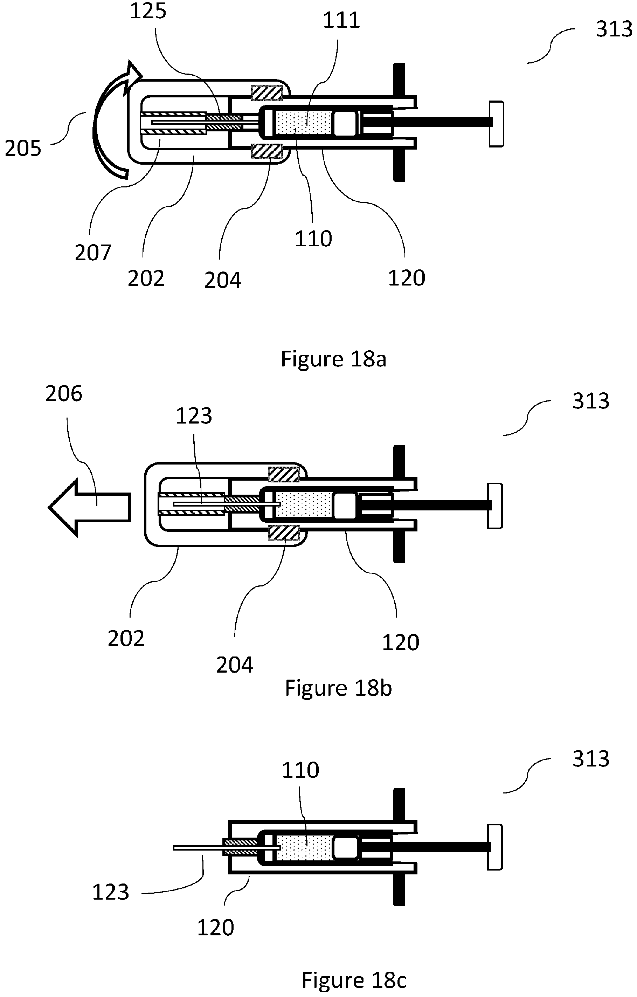

Figures 18 show an alternative embodiment of the invention where a syringe system 313 has a needle cap 202 with a shoulder 207 that engages and pushes needle hub 125 towards the cartridge septum by engaging with a screw thread 204 when rotated by the user as shown by arrow 205 in Figure 18a. Once fully rotated the cap is removed by the user as shown by arrow 206 in Figure 18b. In Figure 18c the syringe system is ready to inject.

An additional needle safety guard may be added to the syringe system 313 shown in Figures 18.

In the syringe system 303 shown in Figures 15, the syringe system 304 shown in Figures 16, the syringe system 311 shown in Figures 17, and the syringe system 313 shown in Figures 18, the cartridge 110 may be held in position in the syringe housing 127 by interference fit between the cartridge barrel and syringe housing or firmly by the cartridge neck or cap at the cartridge distal end and at the cartridge proximal end.

Figures 19 show another embodiment of the invention where the syringe system 314 has a plunger rod 128 attached to a plunger rod guide 603. Plunger rod guide 603 has two or more wings or arms 607 that are flexible or are spring loaded and press against the internal walls of cartridge 110 with sufficient force and creating sufficient friction to keep the plunger rod 128 during transport and handling by the user. At the same time the friction between the plunger rod guide 603 and the cartridge 110 walls is small enough to be easily overcome by the user when pressing plunger rod at pad 129 and pushing the plunger rod against stopper 112 during the mixing, priming or injection stroke. The arrangement and shape of the wings or arms 607 shown in Figures 19b and 19c are only one example of what may be used and many other shapes and arrangements are possible that can achieve the same result.

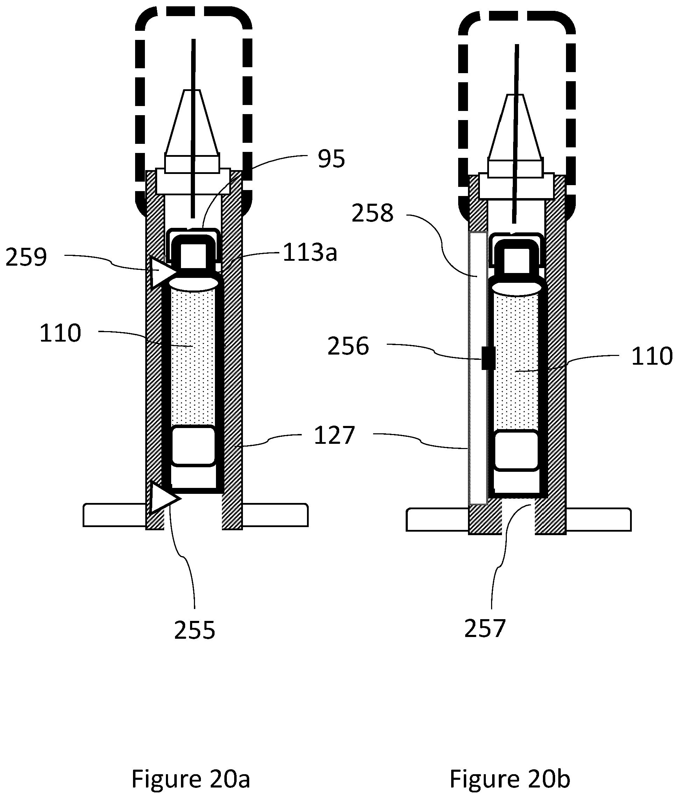

Figures 20 shown alternative ways to insert the cartridge into the syringe housing. In Figure 20a the cartridge 110 is inserted into the syringe housing 127 axially at its proximal end and is held either at the cartridge proximal end in position by one or more snap fits 255 and/or by snap fits 259 at its distal end at the cartridge shoulder (113a) and/or cap (95).

In Figure 20b the cartridge 110 is inserted into the syringe housing 127 radially through a slot 258 and is held in the syringe housing in position by one or more snap fits 256 and by a housing shoulder 257 at the proximal end of the syringe housing.

Note that in the above Figures the injection needle is sometimes shown replaced by nozzle which can be a screw fit, a Luer slip or a Luer lock, a slip tip, a catheter tip or an eccentric tip to enable the user to attach the injection needle or an intravenous infusion tube after removing the device from the sterile packaging. This applies to all embodiments where the delivery means can be a needle or any other means. If a catheter tip or an eccentric tip is supplied, it is advantageous to keep the cap and locking mechanism separate so that the cap can be removed to attach the needle or intravenous line before unlocking the cartridge.

It is also a feature of the present invention that if the syringe system is supplied in a sterile blister or sterile pack a needle or a nozzle cap is not essential. Note that in the above Figures the cartridge may be replaced by a dual chamber cartridge for the reconstitution lyophilized pharmaceuticals or drugs or any other dry powders or the mixing of two pharmaceutical or drug solutions or formulations or any other solutions prior to injection. For mixing the lyophilized drug and the diluent or the two drug formulations the cartridge is pushed forward to perforate the septum allowing the air in the cartridge to exit through the needle when pushing the plunger rod to mix the drug solution. It this arrangement it may be an advantageous to have a two-position needle cap. For example, the cap may be rotated to release the cartridge lock and pulled to remove and remain in place protecting the needle until used. Alternatively in this embodiment it may be advantageous to keep the cap and lock separate so that the cartridge can be unlocked to mix the drug before the cap can be removed and the injection needle exposed. Note that in some embodiments shown in the above Figures the syringe may be supplied to the user already in place or separately. In the Figures the cartridge is shown as supplied already in place or after the user has inserted the cartridge.

The cartridge may be inserted by the user axially from behind in which case the plunger rod may be attached to the syringe body by for instance a hinged or the plunger rod may be detached as a separate part, or the cartridge may be inserted radially from the side through a slot. If the cartridge is supplied to the user already in place in the syringe the combination product is terminally sterilized.

Note that the embodiments of the present invention shown in the Figures may be combined in any way.

## Claims

1. A syringe comprising:
an outer casing or syringe housing (127) with finger pads (131) or finger rings (131a) or a mixture of at the proximal end capable of firmly holding;
a cartridge (110) having a generally tubular glass or plastic barrel (111) with a cap assembly (95) comprising of a needle pierceable septum (114) or sealing element held by a ferrule or crimp (115) at the distal end of the barrel and a stopper/piston (112) disposed at the proximal end of the barrel to contain the medicament (117) or other formulation (117) between the stopper/piston member and the septum or sealing element,
a plunger rod (128) with finger pad (129) or finger ring (129a) at one end and in communication with the cartridge stopper/piston (112) at the other end,
a double needle comprising a septum needle (124) and an injection needle (123), wherein the septum needle (124) projecting from the distal end of the syringe housing (127) into the interior of the syringe housing (127) is rigidly held in the syringe housing (127),
where the cartridge (110) is held axially in a first position within the syringe housing (127) and is prevented from substantially moving unaided axially forwards or backwards within the syringe housing (127) but can be moved axially forward by the user to a second position wherein the cartridge septum (114) is perforated by the septum needle (124), thereby establishing fluid communication between the cartridge contents and the injection needle (123),
wherein the cartridge (110) is held in the first position by a locking mechanism (215) formed as part of the housing (127) or attached to the housing (127), wherein a needle shield cap (450) or a sleeve (450) is used to lock the cartridge (110) at the first position before use, wherein the cap (450), or sleeve (450), respectively, is movable axially (413) relative to the syringe housing (127) to release the locking mechanism (215) so that the cartridge can be pushed to the second position.

2. The syringe according to claim 1, wherein the septum needle (124) and the injection needle (123) both are parts of a single needle body, said single needle body preferably having two sharp, beveled ends.

3. The syringe according to claim 1, wherein said injection needle (123) is separate from said septum needle (124) and said septum needle (124) is in fluid communication with said injection needle (123).

4. The syringe according to any one of the preceding claims, wherein said septum needle (124) is of greater diameter or smaller gauge number than said injection needle (123).

5. The syringe according to any one of the preceding claims where a safety guard (455) with finger flanges (449) or grips is used to lock the cartridge (110) at the first position before use where the sleeve (450) is movable axially (413) relative to the syringe housing (127) to release the locking mechanism (215), wherein the locking mechanism (215) is formed as part of the housing (127) or is attached to the housing (127) and where the lock is held in the locked position by the safety guard (455).

6. The syringe according to any one of the preceding claims where a ring (415) or a needle cap (450) is provided for locking the cartridge (110) at the first position before use, wherein the ring (415) or needle cap (450) is rotatable (415) relative to the outer casing or the syringe housing (127) to release the locking mechanism (214), wherein the locking mechanism is formed as part of the housing (127) or is attached to the housing (127) and where the lock is held in the locked position by the ring (415) or cap (450), respectively.

## Patentansprüche

1. Spritze, aufweisend
einen Spritzenzylinder oder ein Spritzengehäuse (127) mit Fingerauflagen (131) oder Fingerringen (131a) oder einer Mischung davon an dem proximalen Ende, wodurch ein fester Halt ermöglicht wird;
eine Karpule (110), die einen im Allgemeinen rohrförmigen Zylinder (111) aus Glas oder Kunststoff mit einem Kappenaufbau (95), der aus einem mit einer Nadel durchstechbaren Septum (114) oder Versiegelungselement, das durch eine Quetschhülse oder eine Crimpverbindung (115) gehalten wird, besteht, an dem distalen Ende des Zylinders und einem Anschlag/Kolben (112), der an dem proximalen Ende des Zylinders angeordnet ist, aufweist und zwischen dem Anschlag/Kolbenelement und dem Septum oder dem Versiegelungselement ein Arzneimittel (117) oder eine andere Formulierung (117) enthält,
eine Kolbenstange (128) mit einer Fingerauflage (129) oder einem Fingerring (129a) an einem Ende, die an dem anderen Ende mit dem Anschlag/Kolben (112) der Karpule in Verbindung steht,
eine Doppelnadel, die eine Septumnadel (124) und eine Injektionsnadel (123) aufweist, wobei die Septumnadel (124), die von dem distalen Ende des Spritzengehäuses (127) in das Innere des Spritzengehäuses (127) ragt, fest in dem Spritzengehäuse (127) gehalten wird,
wobei die Karpule (110) in der Achsenrichtung an einer ersten Position in dem Spritzengehäuse (127) gehalten wird und an einer nicht unterstützten wesentlichen Vorwärts- oder Rückwärtsbewegung in der Achsenrichtung in dem Spritzengehäuse (127) gehindert wird, aber durch den Benutzer in der Achsenrichtung vorwärts an eine zweite Position bewegt werden kann, wobei das Karpulenseptum (114) durch die Septumnadel (124) durchstochen wird, wodurch eine Fließverbindung zwischen dem Karpuleninhalt und der Injektionsnadel (123) hergestellt wird, wobei die Karpule (110) durch einen Arretiermechanismus (215), der als Teil des Gehäuses (127) ausgebildet ist oder an dem Gehäuse (127) angebracht ist, an der ersten Position gehalten wird, wobei eine Nadelschutzkappe (450) oder eine Hülse (450) verwendet wird, um die Karpule (110) vor der Verwendung an der ersten Position zu arretieren, wobei die Kappe (450) beziehungsweise die Hülse (450) in der Achsenrichtung in Bezug auf das Spritzengehäuse (127) beweglich ist (413), um den Arretiermechanismus (215) zu lösen, so dass die Karpule an die zweite Position geschoben werden kann.

2. Spritze nach Anspruch 1, wobei sowohl die Septumnadel (124) als auch die Injektionsnadel (123) jeweils ein Teil eines einzelnen Nadelkörpers ist, wobei der einzelne Nadelkörper vorzugsweise zwei scharfe, abgeschrägte Enden aufweist.

3. Spritze nach Anspruch 1, wobei die Injektionsnadel (123) von der Septumnadel (124) getrennt ist und die Septumnadel (124) in einer Fließverbindung mit der Injektionsnadel (123) steht.

4. Spritze nach einem der vorhergehenden Ansprüche, wobei die Septumnadel (124) einen größeren Durchmesser oder eine kleinere Gauge-Zahl als die Injektionsnadel (123) aufweist.

5. Spritze nach einem der vorhergehenden Ansprüche, wobei eine Schutzeinrichtung (455) mit Fingerflanschen (449) oder Griffen verwendet wird, um die Karpule (110) vor der Verwendung an der ersten Position zu arretieren, wobei die Hülse (450) in Bezug auf das Spritzengehäuse (127) in der Achsenrichtung beweglich ist (413), um den Arretiermechanismus (215) zu lösen, wobei der Arretiermechanismus (215) als Teil des Gehäuses (127) ausgebildet ist oder an dem Gehäuse (127) angebracht ist, und wobei die Arretierung durch die Schutzeinrichtung (455) in der arretierten Stellung gehalten wird.

6. Spritze nach einem der vorhergehenden Ansprüche, wobei ein Ring (415) oder eine Nadelkappe (450) bereitgestellt ist, um die Karpule (110) vor der Verwendung an der ersten Position zu arretieren, wobei der Ring (415) oder die Nadelkappe (450) in Bezug auf den Spritzenzylinder oder das Spritzengehäuse (127) drehbar ist (415), um den Arretiermechanismus (214) zu lösen, wobei der Arretiermechanismus als Teil des Gehäuses (127) ausgebildet ist oder an dem Gehäuse (127) angebracht ist, und wobei die Arretierung durch den Ring (415) beziehungsweise die Kappe (450) in der arretierten Stellung gehalten wird.

## Revendications

1. Seringue comprenant :
une enveloppe extérieure ou un boîtier de seringue (127) avec des coussinets (131) ou des bagues (131a) pour les doigts ou un mélange de ces éléments situés à l'extrémité proximale et capables de tenir fermement ;
une cartouche (110) ayant un cylindre globalement tubulaire en verre ou en plastique (111) avec un ensemble capuchon (95) comprenant un septum perforable (114) ou un élément d'étanchéité maintenu par une virole ou un sertissage (115) à l'extrémité distale du cylindre et un bouchon/piston (112) disposé à l'extrémité proximale du cylindre pour contenir le médicament (117) ou une autre formulation (117) entre l'élément bouchon/piston et le septum ou l'élément d'étanchéité,
une tige de piston (128) avec un coussinet (129) ou une bague (129a) pour les doigts à une extrémité et en communication avec le piston/bouchon de cartouche (112) à l'autre extrémité,
une aiguille double comprenant une aiguille à septum (124) et une aiguille d'injection (123), l'aiguille à septum (124) faisant saillie de l'extrémité distale du boîtier de seringue (127) à l'intérieur du boîtier de seringue (127) étant maintenue rigidement dans le boîtier de seringue (127),
la cartouche (110) étant maintenue axialement dans une première position à l'intérieur du boîtier de seringue (127) et ne pouvant se déplacer sensiblement sans aide axialement vers l'avant ou vers l'arrière à l'intérieur du boîtier de seringue (127), mais pouvant être déplacée axialement vers l'avant par l'utilisateur dans une seconde position dans laquelle le septum de cartouche (114) est perforé par l'aiguille à septum (124), en établissant ainsi une communication fluidique entre le contenu de la cartouche et l'aiguille d'injection (123),
on la cartouche (110) étant maintenue dans la première position par un mécanisme de verrouillage (215) faisant partie du boîtier (127) ou fixé au boîtier (127), un capuchon protecteur d'aiguille (450) ou un manchon (450) étant utilisé pour verrouiller la cartouche (110) dans la première position avant utilisation, le capuchon (450) ou manchon (450), respectivement, étant mobile axialement (413) par rapport au boîtier de seringue (127) pour libérer le mécanisme de verrouillage (215) de sorte que la cartouche puisse être poussée vers la seconde position.

2. Seringue selon la revendication 1, dans laquelle l'aiguille à septum (124) et l'aiguille d'injection (123) sont toutes deux des parties d'un boîtier d'aiguille unique, ledit boîtier d'aiguille unique ayant de préférence deux extrémités biseautées aiguisées.

3. Seringue selon la revendication 1, dans laquelle ladite aiguille d'injection (123) est séparée de ladite aiguille à septum (124) et ladite aiguille à septum (124) est en communication fluidique avec ladite aiguille d'injection (123).

4. Seringue selon l'une quelconque des revendications précédentes, dans laquelle ladite aiguille à septum (124) est d'un diamètre supérieur ou d'un numéro de jauge inférieur à celui de ladite aiguille d'injection (123).

5. Seringue selon l'une quelconque des revendications précédentes, dans laquelle une protection de sécurité (455) avec des brides de doigt (449) ou des poignées est utilisée pour verrouiller la cartouche (110) dans la première position avant utilisation où le manchon (450) est mobile axialement (413) par rapport au boîtier de seringue (127) pour libérer le mécanisme de verrouillage (215), le mécanisme de verrouillage (215) faisant partie du boîtier (127) ou étant fixé au boîtier (127) et le verrou étant maintenu en position verrouillée par la protection de sécurité (455).

6. Seringue selon l'une quelconque des revendications précédentes, dans laquelle une bague (415) ou un capuchon d'aiguille (450) est prévu pour verrouiller la cartouche (110) dans la première position avant utilisation, la bague (415) ou le capuchon d'aiguille (450) pouvant tourner (415) par rapport à l'enveloppe extérieure ou au boîtier de seringue (127) pour libérer le mécanisme de verrouillage (214), le mécanisme de verrouillage faisant partie du boîtier (127) ou étant fixé au boîtier (127) et le verrou étant maintenu en position verrouillée par la bague (415) ou le capuchon (450), respectivement.
